# EUROPÄISCHE PATENTSCHRIFT

(11) **EP 3 822 618 B1**
(45) Veröffentlichungstag und Bekanntmachung des Hinweises auf die Patenterteilung: **11.09.2024**
(21) Anmeldenummer: 20205264.3
(22) Anmeldetag: 02.11.2020
(51) Int. Cl.: G01N 21/85, G01N 21/25, G01N 21/94, G01N 33/02, G01N 33/04, B07C 5/342

(54) **VERFAHREN UND VORRICHTUNG ZUR ERKENNUNG VON FREMDKÖRPERN**
METHOD AND DEVICE FOR DETECTING FOREIGN BODIES
PROCÉDÉ ET DISPOSITIF DE DÉTECTION DES CORPS ÉTRANGERS

(30) Priorität: 15.11.2019 DE 102019130883
(43) Veröffentlichungstag der Anmeldung: 19.05.2021
(73) Patentinhaber: SWAT Automation GmbH, 01945 Senftenberg OT Peickwitz (DE)
(72) Erfinder: Jurisch, Michael, 01945 Senftenberg OT Peickwitz (DE); Gorte, Maxim, 01945 Senftenberg OT Peickwitz (DE)
(74) Vertreter: Werner, André

(56) Entgegenhaltungen:
- EP-A1- 1 582 272
- EP-A2- 0 719 598
- CN-A- 103 170 462
- GB-A- 2 534 753
- US-A1- 2011 317 001

## Beschreibung

Die vorliegende Erfindung betrifft ein Verfahren und eine Vorrichtung zum Erkennen von Fremdkörpern in einem Schüttgut, z. B. Lebensmittel, das in einem Materialstrom durch einen Detektionsbereich gefördert wird.

In der Lebensmittelproduktion ist das Auftreten von Fremdkörpern in den Lebensmitteln ein nicht unerhebliches Problem hinsichtlich Produktqualität und Verbraucherschutz. Zur Vermeidung von Fremdkörpern in den Lebensmitteln gibt es zahlreiche Ansätze. Neben präventiver Maßnahmen, die eine Verunreinigung der Lebensmittel mit Fremdkörpern vermeiden helfen sollen, gibt es Verfahren zur gezielten Aussortierung, z. B. mittels Filtern, Zentrifugen, Druckluftdüsen oder Sieben, sowie zur systematischen Erkennung der Fremdkörper im Lebensmittel.

Aus der DE 10 2018 121 762 A1 ist bekannt, einen Metalldetektor zu verwenden, der einen Detektionsbereich aufweist, durch den Lebensmittel gefördert werden. In einer Auswerteeinrichtung des Metalldetektors werden Fremdkörpern als metallhaltig detektiert, wenn das erhaltene Messsignal wenigstens einen Referenzwert überschreitet.

Nachteil der auf Metalldetektoren basierenden Verfahren ist ihre Beschränkung einzig auf metallische bzw. metallhaltige Fremdkörper. Beispielsweise werden Kunststofffolien hierdurch nicht erfasst.

EP 3 028 031 B1 beschreibt ein Verfahren zur Detektion von Fremdkörpern in Behältern, die eine Behälterwand aus einem optisch transparenten Material aufweisen. Hierbei werden mittels optischer Kohärenztomographie (OCT) tomographische Daten wenigstens eines Volumenbereiches im Inneren der Behälter aufgezeichnet. Zwar werden mit diesem Verfahren Fremdkörper bzw. Fremdkörperpartikel weitgehend unabhängig von ihren optischen Eigenschaften erfasst, doch ist das Verfahren nur bei optisch transparenten Produkten, z. B. Flüssigkeiten, anwendbar.

Die in DE 10 2011 075 607 A1 gezeigte Verpackungsvorrichtung mit Fremdkörpererfassung umfasst eine Dosierschnecke, welche das Produkt einer Verpackung zuführt. An der Dosierschnecke sind zwei Ultrabreitbandsensoren angeordnet, mittels derer kontinuierlich eine Dichte des Produkts detektiert wird. Ein außerhalb einer vorgegebenen Bandbreite liegender Dichtewert führt zum Ausscheiden der Verpackung. Allerdings werden Fremdkörper mit einer zum Produkt ähnlichen Dichte hierdurch nicht erkannt.

Die DE 10 2017 124 895 B3 beschreibt eine Anordnung zur Fremdkörpererkennung in einer Mehrkopfwaage, die in einen Trichter entleerbar ist. Die Mehrkopfwaage weist eine Kamera zur Aufnahme einer Bildserie am Trichter, eine Beleuchtungseinheit und eine Bildauswerteeinheit zur Abgabe eines Signals bei einer Fremdkörperdetektion in einer Bildserie auf. Durch die Integration der Anordnung zur Fremdkörpererkennung in die Mehrkopfwaage ist eine - insbesondere im Lebensmittelbereich vorgeschriebene - regelmäßige Reinigung erschwert. Außerdem werden in der Mehrkopfwaage gesammelte, saubere Chargen zuerst mit den verschmutzten Chargen gemischt, bevor sie ausgeschleust werden.

DE 20 2006 016 604 U1 zeigt eine Vorrichtung zur optischen Selektion von Bestandteilen wenigstens einer Fraktion aus einem längs einer Förderrichtung geförderten Schüttgutstrom, mit einer Kameraeinheit, deren Blickrichtung auf den Schüttgutstrom gerichtet ist, einem in Kamerablickrichtung hinter dem Schüttgutstrom angeordneten, aktiv leuchtenden Hintergrund, dessen Farbe an die Farbe einer ausgewählten Fraktion von Bestandteilen des Schüttgutstromes anpassbar ist, eine mit der Kameraeinheit in Verbindung stehende Auswerte- und Steuereinheit, in der nach einem Entscheidungskriterium Steuerbefehle für eine Separiereinheit generiert werden, die bei Ansteuerung die zu selektierenden Bestandteile aus dem Schüttgutstrom zu separieren vermag.

EP 1 498 723 A1 offenbart ein Verfahren und eine Vorrichtung zum Auffinden von Fremdkörpern in Tabak. Hierbei wird der kontinuierlich auf einem Förderband geführte Tabakstrom mit einem aufgefächerten Laserstrahl linienförmig beleuchtet und das reflektierte Licht mit einer Kamera erfasst.

GB 2 534 753 A zeigt ein Beleuchtungssystem für eine Getreidesortiermaschine, wobei die Getreidekörner von einem Rüttler in eine Rinne fallen und von dort durch einen beleuchteten Detektionsbereich fallen. Kameras sind auf den Detektionsbereich bzw. dahinter angeordnete Reflexionselemente gerichtet und erfassen Bilder des fallenden Schüttguts. Die Beleuchtungseinrichtungen beleuchten sowohl das Schüttgut als auch die Reflexionselemente.

US 2011/317001 A1 betrifft ein Sensorfeld für eine Sortiermaschine. Jeder Sensor des Sensorfelds beinhaltet eine Beleuchtungseinrichtung und eine Kamera. Die Partikel, hier Holzschnipsel oder Baumwollfasern, fliegen beschleunigt durch einen Detektionsbereich, der von einem Hintergrund und dem Sensorfeld begrenzt wird. CN 103 170 462 A beschreibt ein Verfahren und eine Vorrichtung zur Sortierung von Schüttgut, bei welchen das von einer Lichtquelle emittierte Licht zweifach durch das Schüttgut läuft.

EP 0 719 598 A2 offenbart eine Farbsortiervorrichtung, die eine Kornführungsvorrichtung, eine Beleuchtungsvorrichtung zum Beleuchten des Korns, einen optischen Detektor sowie einen Auswerfer zum Zurückweisen des Korns umfasst. Die Beleuchtungsvorrichtung verwendet mindestens eine Lichtquelle mit einer spektralen Energieverteilung sowohl im sichtbaren Lichtbereich als auch im nahen Infrarotbereich.

EP 1 582 272 A1 betrifft ein Verfahren zur Fremdkörperabscheidung und eine Vorrichtung zur Fremdkörperabscheidung aus einem Tabakstrom, der den Bereich einer Fremdkörpererkennungsvorrichtung mit Druckluft gefördert wird.

Der Erfindung liegt die Aufgabe zugrunde, die oben genannten Nachteile, wie die Beschränkung auf Fremdkörper eines dedizierten Materials, zu vermeiden, wobei ein Verfahren und eine Vorrichtung zur Erkennung von Fremdkörpern in einem Förderstrom eines Schüttguts z. B. in einer Abfüllanlage realisierbar sein soll, die eine im Abfüllprozess frühzeitige (und somit kosteneffiziente) Fremdkörpererkennung unabhängig vom Material der Fremdkörper sowie eine ungehinderte Reinigung der Abfüllanlage ermöglichen.

Die Aufgabe wird durch ein Verfahren zur Erkennung von Fremdkörpern in einem Förderstrom eines Schüttguts mit den kennzeichnenden Merkmalen nach Patentanspruch 1 und eine Vorrichtung zur Erkennung von Fremdkörpern in einem Förderstrom eines Schüttguts mit den kennzeichnenden Merkmalen nach Patentanspruch 4 gelöst. Zweckmäßige Ausgestaltungen der Erfindung finden sich in den abhängigen Ansprüchen.

Das Verfahren gemäß der Offenbarung sieht vor, den mittels einer Fördervorrichtung transportierten Förderstrom von Schüttgutkörpern vor einer Abbruchkante der Fördervorrichtung zu verbreitern und in seiner Höhe zu begrenzen, bevor die Schüttgutkörper durch einen mittels Beleuchtungseinrichtungen beleuchteten, direkt unterhalb der Abbruchkante des Förderstroms angeordneten Detektionsbereich fallen. Der derart begrenzte Schüttgutkörperstrom fällt an der Abbruchkante der Fördervorrichtung in eine Abfüllanlage, z. B. eine Mehrkopfwaage, oder auf ein abfahrfähiges Transportband.

Auf den direkt unterhalb der Abbruchkante angeordneten, mittels Beleuchtungseinrichtungen beleuchteten Detektionsbereich, durch den die zuvor quasi vereinzelten Schüttgutkörper fallen, ist wenigstens eine Kamera gerichtet. Der wenigstens einen Kamera bezüglich des Detektionsbereichs gegenüberliegend ist ein flächig ausgebildetes Reflexionselement angeordnet, d. h., die Schüttgutkörper fallen zwischen Reflexionselement und der wenigstens einen Kamera durch den Detektionsbereich. Von den fallenden Schüttgutkörpern wird von der wenigstens einen Kamera in vorgegebenen Zeitabständen jeweils ein Bild erfasst, dass mittels Bildauswertung hinsichtlich Farbe und/oder Kontur der abgebildeten Körper analysiert wird.

Falls eine von der Farbe und/oder Kontur der Schüttgutkörper abweichende Farbe bzw. Kontur in einem der Bilder detektiert wird, wird ein Signal generiert, z. B. ein Alarm- und/oder Stoppsignal. Zusätzlich wird das fragliche Bild in einem Bildspeicher zusammen mit weiteren Prozessdaten, z. B. Datum und Uhrzeit, abgelegt. Zusätzlich kann ein Bildschirm das aktuell letzte Bild mit Fremdköpererkennung für den Bediener anzeigen.

Die Bildaufnahmefrequenz ist vergleichsweise hoch; sie beträgt wenigstens 40 Bilder pro Sekunde, bevorzugt über 50 Bilder pro Sekunde. Die Bildaufnahme erfolgt im Endlosbetrieb, somit kann eine dauerhafte, unterbrechungsfreie Detektion gewährleistet werden.

Es kann vorgesehen sein, dass im Falle eines Signals, d. h., wenn die Bilderfassung einen Fremdkörper detektiert, das betroffene Schüttgut - vorzugsweise automatisiert - aussortiert wird, bevor es in die Abfüllanlage fällt. Das Aussortieren kann mittels eines abfahrfähigen Transportbandes, welches das verunreinigte Schüttgut z. B. entgegen der Produktionsrichtung in ein Abfallgefäß bzw. in Produktionsrichtung mittels Umklappens in ein Abfallgefäß abführt, erfolgen.

In vorteilhafter Weise kann das Verfahren verwendet werden, um geriebenen oder gewürfelten Käse, der auch mit einem Trennmittel, z. B. Stärke, behandelt sein kann, vor dem Abwiegen und Abfüllen in Verpackungseinheiten auf Fremdkörper, z. B. Kunststofffolienteilchen, hin zu überwachen.

Die offenbarungsgemäße Vorrichtung zur Erkennung von Fremdkörpern in einem Förderstrom eines Schüttguts umfasst ein flächig ausgebildetes Reflexionselement sowie wenigstens eine auf dasselbe gerichtete Kamera, wobei sich zwischen dem Reflexionselement und der wenigstens einen Kamera der Detektionsbereich befindet, durch den das Schüttgut fällt.

Weiterhin umfasst die Vorrichtung drei Beleuchtungseinrichtungen zur Beleuchtung des Detektionsbereichs und des Reflexionselementes. In vorteilhafter Weise beleuchtet jede der Beleuchtungseinrichtungen den Detektionsbereich aus einer anderen Richtung, sodass das fallende Schüttgut von den Beleuchtungseinrichtungen aus drei unterschiedlichen Richtungen beleuchtet wird.

Eine der Beleuchtungseinrichtungen ist auf einer Gehäusewand eines die mindestens eine Kamera umhausenden Gehäuses angeordnet oder Bestandteil derselben. Das Leuchtelement ist hierbei flächig ausgebildet. Zumindest das Gehäuse weist einen transparenten Bereich auf, der als Fenster für die wenigstens eine Kamera dient.

Das Gehäuse kann hierbei leicht nach vorne gekippt sein, d. h., die die Beleuchtungseinrichtung umfassende Gehäusewand ist um wenige Grad, z. B. 5°, zur Lotrechten gekippt.

Die dem Detektionsbereich zugewandte Gehäusewand kann auch vollständig durch eine der Beleuchtungseinrichtungen gebildet sein, wobei in diesem Fall die Beleuchtungseinrichtung wenigstens einen transparenten Bereich aufweist, der als Kamerafenster dient. Insbesondere kann das Gehäuse trotz Integration der Beleuchtungseinrichtung staubdicht ausgebildet sein.

Die Kamera bzw. Kameras sind mit einer Bildauswerteeinrichtung verbunden, die eingerichtet ist, ein Signal zu generieren, wenn innerhalb eines mittels der mindestens einen Kamera erfassten Bildes eine von der Farbe des Schüttguts abweichende Farbe und/oder eine von der Kontur des Schüttguts abweichende Kontur auftritt.

Das hierbei generierte Signal kann ein (akustisches, elektrisches und/oder optisches) Alarmsignal und/oder ein an eine Steuereinheit gerichtetes Steuersignal sein, wobei die Steuereinheit beispielsweise den Förderstrom des Schüttguts unterbricht oder eine Ausschleusungsvorrichtung ansteuert, die den kontaminierten Teil des Förderstroms aus der Anlage ausschleust, z. B. bevor das Fremdkörper-belastete Schüttgut in eine Abfüllanlage mit Mehrkopfwaage fällt.

Die Beleuchtungseinrichtungen emittieren breitbandiges Licht in einem von der wenigstens einen Kamera erfassbaren Wellenlängenbereich, vorzugsweise im sichtbaren Bereich.

Bevorzugt weist das flächige Reflexionselement eine matte Oberfläche in einer dem Schüttgut angepassten Farbe auf. Die Größe, d. h. die Fläche, des Reflexionselementes ist derart gewählt, dass es den gesamten von der wenigstens einen Kamera erfassten Bildausschnitt abdeckt.

Alle Beleuchtungseinrichtungen können flächig ausgebildet sein; sie sind auf derselben Höhe bezüglich des Detektionsbereiches in der Art angeordnet, dass sie den

Detektionsbereich bzw. das durch diesen fallende Schüttgut aus unterschiedlichen Richtungen beleuchten.

Zwei der drei Beleuchtungseinrichtungen sind bezüglich des Detektionsbereiches einander gegenüberliegend angeordnet, sodass sie sich - ohne fallendes Schüttgut - gegenseitig anstrahlen. Auch können die Beleuchtungseinrichtungen eine körperliche Einheit bilden, wobei sie z. B. durch einen Steg verbunden oder als ein U-Förmiges Bauteil ausgebildet sind.

Eine Ausgestaltung sieht vor, dass die Vorrichtung wenigstens zwei Kameras aufweist. Hierbei sind alle Kameras auf das Reflexionselement ausgerichtet, wobei die Blickrichtungen der Kameras auf das Reflexionselement jeweils unterschiedlich sind, d. h. die Blickrichtungen sind in einem Winkel zueinander angeordnet, wobei dieser jeweils maximal 30°, bevorzugt jeweils maximal 10°, beträgt. Insbesondere überschneiden sich die mit den einzelnen Kameras erfassten Bildausschnitte, sodass durch unterschiedliche Blickwinkel auf den Schüttgutstrom eine verbesserte Fremdkörperdetektion ermöglicht ist. Vorzugsweise sind die Kameras auf einer horizontalen Linie bzw. entlang eines horizontalen Bogens angeordnet. Zusätzlich können die Kameras in einem gemeinsamen Gehäuse gekapselt sein.

Weiter kann vorgesehen werden, dass die Vorrichtung eine in Förderrichtung des Schüttguts vor dem Detektionsbereich angeordnete Vereinzelungseinrichtung aufweist, mit der die Dicke des Förderstromes begrenzbar ist, wodurch eine Detektion von Fremdkörpern erleichtert ist.

Zusätzlich oder alternativ kann vorgesehen sein, dass die Vorrichtung oder Teile von ihr mittels einer Verschiebevorrichtung mit nur geringem Kraftaufwand und unter Verzicht auf Werkzeug in zumindest einem Freiheitsgrad, z. B. transversal nach oben, verschiebbar ist. Insbesondere kann die Vorrichtung ein die mindestens eine Kamera umhausendes, staubdichtes Gehäuse aufweisen, welches mittels einer Hebelarme und Gelenke umfassenden Verschiebevorrichtung für z. B. Reinigungszwecke verschoben werden kann. Für einen reduzierten Kraftaufwand beim Verschieben kann die Verschiebevorrichtung auch mechanische und/oder hydraulische Federelemente umfassen.

Gemäß einer Ausführungsform kann das Reflexionselement von einer Beleuchtungseinrichtung direkt beleuchtet sein und/oder selbst ein Beleuchtungselement umfassen, das den Schüttgutstrom beleuchtet.

Die Vorrichtung zur Erkennung von Fremdkörpern ist vorteilhaft einsetzbar in der Lebensmittelindustrie bei der Überwachung von schüttfähigen Lebensmitteln, beispielsweise geriebenem oder gewürfeltem Käse oder Hülsenfrüchten, auf Fremdkörper.

Die Erfindung wird nachfolgend anhand eines Ausführungsbeispiels näher erläutert, wobei gleiche oder ähnliche Merkmale mit gleichen Bezugszeichen versehen sind. Dazu zeigen in schematischer Darstellung die
- Fig. 1:: den prinzipiellen Aufbau einer Vorrichtung zur Erkennung von Fremdkörpern in Reibekäse;
- Fig. 2:: eine Vorrichtung zur Erkennung von Fremdkörpern in Reibekäse gemäß einer Ausgestaltung;
- Fig. 3:: ein Kameragehäuse mit integrierter Beleuchtungseinrichtung;
- Fig. 4:: eine Vorrichtung zur Erkennung von Fremdkörpern in Reibekäse gemäß einer weiteren Ausgestaltung; und
- Fig. 5:: eine Vorrichtung zur Erkennung von Fremdkörpern in einem Schüttgut aus Erbsen gemäß einer Ausgestaltung.

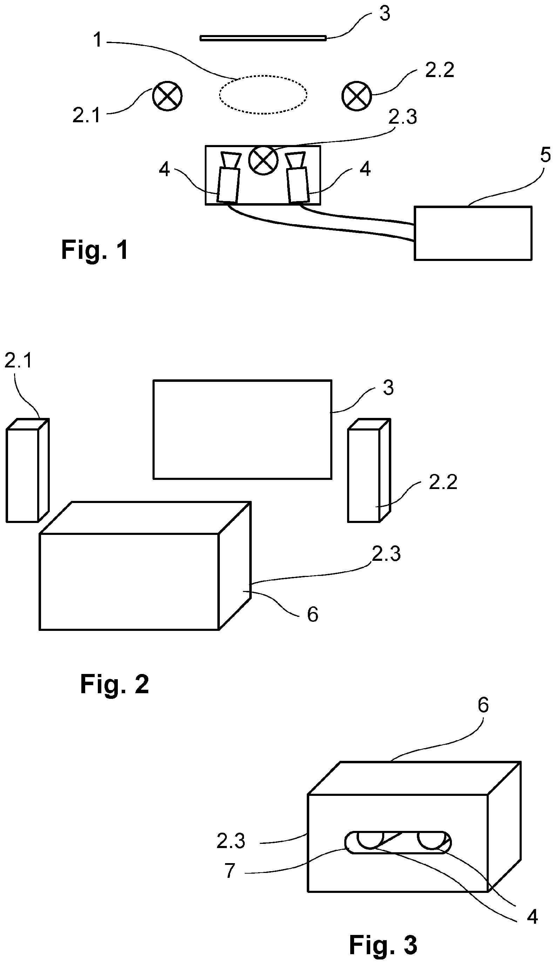

Gemäß Fig. 1 sind rings um den Detektionsbereich 1, durch welchen der Reibekäse (nicht dargestellt) fällt, in einer Ebene (der Papierebene) die Beleuchtungseinrichtungen 2.1, 2.2 und 2.3, das Reflexionselement 3 und die Kameras 4 angeordnet. In diesem Beispiel fällt der Reibekäse von oben in die Papierebene hinein. Die Kameras 4 sind mit der Bildauswerteeinrichtung 5 verbunden. Die Beleuchtungseinrichtung 2.3 und die Kameras 4 bilden eine bauliche Einheit.

In der Ausgestaltung der Vorrichtung gemäß Fig. 2 sind die Kameras (nicht dargestellt) in dem staubdichten Gehäuse 6 aus Edelstahl untergebracht, auf dessen dem Reflexionselement 3 zugewandter Vorderseite die Beleuchtungseinrichtung 2.3 angeordnet ist. Die beiden anderen Beleuchtungseinrichtungen 2.1 und 2.2 sind - analog zu Fig. 1 - jeweils links und rechts des Detektionsbereiches 1 angeordnet.

Fig. 3 zeigt das Gehäuse 6 von seiner Vorderseite. Die großflächig ausgebildete Beleuchtungseinrichtung 2.3 bedeckt die gesamte Vorderseite mit Ausnahme einer schlitzförmigen Ausnehmung, die als Kamerafenster 7 dient, sodass die zwei Kameras 4 freie Sicht auf den gut beleuchteten Schüttgutstrom von Reibekäse (nicht dargestellt) haben. Die transparente Vorderseite des Gehäuses 6 besteht aus Makrolon.

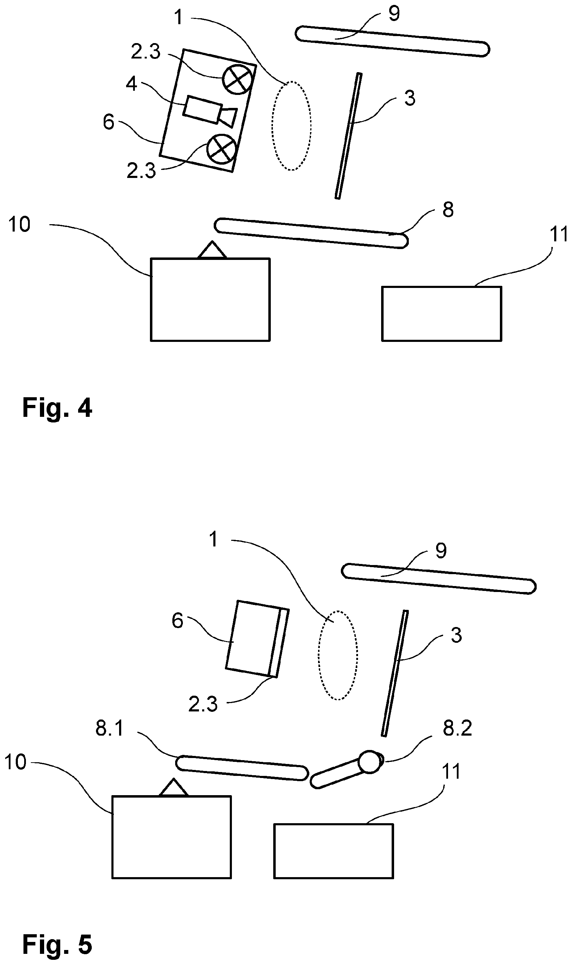

Die Vorrichtung zur Erkennung von Fremdkörpern in Reibekäse gemäß der Ausgestaltung in Fig. 4 umfasst das Abführband 8, welches den vom Zuführband 9 durch den Detektionsbereich 1 fallenden Reibekäse (nicht dargestellt) in die Mehrkopfwaage 10 zum Verpacken des "guten" Reibekäses oder alternativ (durch Bewegungsumkehr in die entgegengesetzte Richtung) in den Abfallbehälter 11 zur Aussortierung des "verunreinigten" Käses transportiert. Die Beleuchtungseinrichtung 2.3 und die Kamera 4 bilden eine bauliche Einheit, indem sie in dem Gehäuse 6 angeordnet sind.

Die Vorrichtung zur Erkennung von Fremdkörpern beispielsweise zwischen Erbsen gemäß Fig. 5 umfasst die beiden Abführbänder 8.1 und 8.2, wobei das zweite Abführband 8.2 kippbar ausgestaltet ist. Bei Detektion eines Fremdkörpers in den durch den Detektionsbereich fallenden Erbsen (nicht dargestellt) wird das zweite Abführband 8.2 nach unten gekippt, sodass die "verunreinigten" Erbsen in den Abfallbehälter 11 fallen.

### Liste der verwendeten Bezugszeichen

- 1: Detektionsbereich
- 2.1: erste Beleuchtungseinrichtung
- 2.2: zweite Beleuchtungseinrichtung
- 2.3: dritte Beleuchtungseinrichtung
- 3: Reflexionselement
- 4: Kamera
- 5: Bildauswerteeinrichtung
- 6: Gehäuse
- 7: Kamerafenster
- 8: Abführband
- 8.1: Abführband
- 8.2: Abführband, kippbar
- 9: Zuführband
- 10: Mehrkopfwaage
- 11: Abfallbehälter

## Patentansprüche

1. Verfahren zur Erkennung von Fremdkörpern in einem Förderstrom eines Schüttguts, wobei der Förderstrom von Schüttgutkörpern mittels einer Fördervorrichtung transportiert wird und die Schüttgutkörper durch einen mittels drei Beleuchtungseinrichtungen (2.1, 2.2, 2.3) beleuchteten Detektionsbereich (1) fallen,
wobei
- der Förderstrom von Schüttgutkörpern vor einer Abbruchkante der Fördervorrichtung verbreitert und in seiner Höhe begrenzt wird, bevor die Schüttgutkörper durch den direkt unterhalb der Abbruchkante des Förderstroms angeordneten Detektionsbereich (1) fallen, und
- mittels wenigstens einer Kamera (4) in vorgegebenen Zeitabständen jeweils ein Bild des vor einem flächig ausgebildeten Reflexionselement (3) fallenden Schüttguts erfasst, die erfassten Bilder hinsichtlich Farbe und/oder Kontur der Schüttgutkörper ausgewertet und auf einem Datenträger gespeichert werden,
**dadurch gekennzeichnet, dass**
- die drei Beleuchtungseinrichtungen (2.1, 2.2, 2.3) in einer Ebene auf derselben Höhe bezüglich des Detektionsbereiches (1) in der Art angeordnet sind, dass sie den Detektionsbereich (1) aus unterschiedlichen Richtungen beleuchten, wobei
- von den drei Beleuchtungseinrichtungen (2.1, 2.2, 2.3) eine Beleuchtungseinrichtung (2.3) mit der mindestens einen Kamera (4) eine bauliche Einheit bildet,
- die mit der Kamera (4) eine bauliche Einheit bildende Beleuchtungseinrichtung (2.3) dem flächigen Reflexionselement (3) zugewandt ist, und
- die beiden anderen Beleuchtungseinrichtungen (2.1, 2.2) bezüglich des Detektionsbereichs (1) einander gegenüberliegend angeordnet sind, sodass sie sich gegenseitig anstrahlen.

2. Verfahren zur Erkennung von Fremdkörpern nach Anspruch 1, **gekennzeichnet dadurch, dass** das Schüttgut ausgeschleust wird, wenn die erfassten Bilder des Schüttguts hinsichtlich Farbe und/oder Kontur der Schüttgutkörper abweichende Fremdkörper zeigen.

3. Verfahren zur Erkennung von Fremdkörpern nach Anspruch 1, **gekennzeichnet dadurch, dass** geriebener und mit einem Trennmittel beschichteter Käse als Schüttgut verwendet wird.

4. Vorrichtung zur Erkennung von Fremdkörpern in einem Förderstrom eines fallenden Schüttguts, aufweisend einen von einem Materialstrom fallend durchströmbaren Detektionsbereich (1) sowie mindestens eine auf den Detektionsbereich (1) gerichtete Kamera (4), **gekennzeichnet dadurch, dass**
der Detektionsbereich (1) von drei Beleuchtungseinrichtungen (2.1, 2.2, 2.3) und einem flächigen Reflexionselement (3) berandet ist,
wobei von den drei Beleuchtungseinrichtungen (2.1, 2.2, 2.3) eine Beleuchtungseinrichtung (2.3) mit der mindestens einen Kamera (4) eine bauliche Einheit bildet, wobei die mit der Kamera (4) eine bauliche Einheit bildende Beleuchtungseinrichtung (2.3) ein flächig ausgebildetes Leuchtelement umfasst, welches auf einer Gehäusewand eines die mindestens eine Kamera (4) umhausenden Gehäuses (6) angeordnet oder Bestandteil derselben ist, und wobei die mit der Kamera (4) eine bauliche Einheit bildende Beleuchtungseinrichtung (2.3) auf der dem flächigen Reflexionselement (3) zugewandten Vorderseite des Gehäuses (6) angeordnet ist, wobei das flächig ausgebildete Leuchtelement einen als Kamerafenster (7) ausgebildeten transparenten Bereich aufweist,
wobei die drei Beleuchtungseinrichtungen (2.1, 2.2, 2.3) in einer Ebene auf derselben Höhe bezüglich des Detektionsbereiches (1) in der Art angeordnet sind, dass sie den Detektionsbereich (1) aus unterschiedlichen Richtungen beleuchten,
wobei die mindestens eine mit einer Bildauswerteeinrichtung (5) verbundene Kamera (4) auf das hinter dem Detektionsbereich (1) angeordnete Reflexionselement (3) gerichtet ist, wobei das flächig ausgebildete Reflexionselement (3) der wenigstens einen Kamera sowie die mit der Kamera (4) eine bauliche Einheit bildendenden Beleuchtungseinrichtung (2.3) bezüglich des Detektionsbereichs (1) gegenüberliegend angeordnet ist, und wobei die beiden anderen Beleuchtungseinrichtungen (2.1, 2.2) bezüglich des Detektionsbereichs (1) einander gegenüberliegend angeordnet sind, sodass sie sich gegenseitig anstrahlen,
wobei die Bildauswerteeinrichtung (5) eingerichtet ist ein Signal zu generieren, wenn innerhalb eines mittels der mindestens einen Kamera (4) erfassten Bildes eine von der Farbe des Schüttguts abweichende Farbe und/oder eine von der Kontur des Schüttguts abweichende Kontur auftritt.

5. Vorrichtung zur Erkennung von Fremdkörpern nach Anspruch 4, **gekennzeichnet dadurch, dass** das flächige Reflexionselement (3) eine matte Oberfläche in der Farbe des Schüttguts aufweist.

6. Vorrichtung zur Erkennung von Fremdkörpern nach Anspruch 4, **gekennzeichnet dadurch, dass** eine erste Beleuchtungseinrichtung (2.1) und eine zweite Beleuchtungseinrichtung (2.2) bezüglich des Detektionsbereichs (1) einander gegenüberliegend angeordnet sind.

7. Vorrichtung zur Erkennung von Fremdkörpern nach Anspruch 4, **gekennzeichnet dadurch, dass** sie wenigstens zwei Kameras (4) aufweist, wobei ein Winkel zwischen den Blickrichtungen der Kameras (4) auf den Detektionsbereich (1) maximal 30° beträgt, wobei die Kameras (4) und das Reflexionselement (3) bezüglich des Detektionsbereichs (1) einander gegenüberliegend angeordnet sind.

8. Vorrichtung zur Erkennung von Fremdkörpern nach Anspruch 4, **gekennzeichnet dadurch, dass** sie eine in Förderrichtung des Schüttguts vor dem Detektionsbereich (1) angeordnete Vereinzelungseinrichtung aufweist, mit der die Dicke des Förderstromes begrenzbar ist.

9. Vorrichtung zur Erkennung von Fremdkörpern nach Anspruch 4, **gekennzeichnet dadurch, dass** das die mindestens eine Kamera (4) umhausende Gehäuse (6) staubdicht ist, wobei es mittels einer Hebelarme und Gelenke umfassenden Verschiebevorrichtung zumindest in einem Freiheitsgrad verschiebbar ist.

## Claims

1. Method for detecting foreign bodies in a conveying flow of bulk material, wherein the conveying flow of bulk material bodies is transported by means of a conveying device and the bulk material bodies fall through a detection area (1) illuminated by means of three lighting devices (2.1, 2.2, 2.3),
wherein
- the conveying flow of bulk material bodies is widened in front of a break-off edge of the conveying device and its height is limited before the bulk material bodies fall through the detection area (1) arranged directly below the break-off edge of the conveying flow, and
- by means of at least one camera (4) an image of the bulk material falling in front of a flat reflection element (3) is captured at predetermined time intervals, the captured images are evaluated with regard to color and/or contour of the bulk material bodies and stored on a data carrier,
**characterized in that**
- the three lighting devices (2.1, 2.2, 2.3) are arranged in a plane at the same height with respect to the detection area (1) in such a way that they illuminate the detection area (1) from different directions, wherein
- of the three lighting devices (2.1, 2.2, 2.3), one lighting device (2.3) forms a structural unit with the at least one camera (4),
- the lighting device (2.3) forming a structural unit with the camera (4) faces the flat reflection element (3), and
- the two other illumination devices (2.1, 2.2) are arranged opposite one another with respect to the detection area (1) so that they illuminate one another.

2. Method for detecting foreign bodies according to claim 1, **characterized in that** the bulk material is discharged if the captured images of the bulk material show foreign bodies that differ in color and/or contour of the bulk material bodies.

3. Method for detecting foreign bodies according to claim 1, **characterized in that** grated cheese coated with a release agent is used as bulk material.

4. Device for detecting foreign bodies in a conveying flow of a falling bulk material, comprising a detection area (1) through which a falling material flow can flow and at least one camera (4) directed at the detection area (1), **characterized in that**
the detection area (1) is bordered by three lighting devices (2.1, 2.2, 2.3) and a flat reflection element (3),
wherein of the three lighting devices (2.1, 2.2, 2.3), one lighting device (2.3) forms a structural unit with the at least one camera (4), wherein the lighting device (2.3) forming a structural unit with the camera (4) comprises a flat lighting element which is arranged on a housing wall of a housing (6) enclosing the at least one camera (4) or is a component thereof, and wherein the lighting device (2.3) forming a structural unit with the camera (4) is arranged on the front side of the housing (6) facing the flat reflection element (3), wherein the flat lighting element has a transparent area designed as a camera window (7),
wherein the three lighting devices (2.1, 2.2, 2.3) are arranged in a plane at the same height with respect to the detection area (1) in such a way that they illuminate the detection area (1) from different directions,
wherein the at least one camera (4) connected to an image evaluation device (5) is directed at the reflection element (3) arranged behind the detection area (1), wherein the flat reflection element (3) of the at least one camera and the lighting device (2.3) forming a structural unit with the camera (4) are arranged opposite one another with respect to the detection area (1), and wherein the two other illumination devices (2.1, 2.2) are arranged opposite one another with respect to the detection area (1) so that they illuminate one another,
wherein the image evaluation device (5) is designed to generate a signal when a color deviating from the color of the bulk material and/or a contour deviating from the contour of the bulk material occurs within an image captured by means of the at least one camera (4).

5. Device for detecting foreign bodies according to claim 4, **characterized in that** the flat reflection element (3) has a matt surface in the color of the bulk material.

6. Device for detecting foreign bodies according to claim 4, **characterized in that** a first lighting device (2.1) and a second lighting device (2.2) are arranged opposite one another with respect to the detection area (1).

7. Device for detecting foreign bodies according to claim 4, **characterized in that** it has at least two cameras (4), wherein an angle between the viewing directions of the cameras (4) on the detection area (1) is a maximum of 30°, wherein the cameras (4) and the reflection element (3) are arranged opposite one another with respect to the detection area (1).

8. Device for detecting foreign bodies according to claim 4, **characterized in that** it has a separating device arranged in the conveying direction of the bulk material in front of the detection area (1), with which the thickness of the conveying flow can be limited.

9. Device for detecting foreign bodies according to claim 4, **characterized in that** the housing (6) surrounding the at least one camera (4) is dust-tight, wherein it is displaceable in at least one degree of freedom by means of a displacement device comprising lever arms and hinges.

## Revendications

1. Procédé de détection des corps étrangers dans un flux de transport d'un produit en vrac, le flux de transport de corps de produits en vrac étant transporté au moyen d'un dispositif de transport et les corps de produits en vrac tombant à travers une zone de détection (1) éclairée au moyen de trois dispositifs d'éclairage (2.1, 2.2, 2.3), dans lequel
- le flux de transport de corps en vrac est élargi devant un bord d'interruption du dispositif de transport et est limité en hauteur avant que les corps de produits en vrac ne tombent à travers la zone de détection (1) disposée directement en dessous du bord d'interruption du flux de transport, et
- au moyen d'au moins une caméra (4), une image du produit en vrac tombant devant un élément de réflexion (3) réalisé de manière plane est saisie à des intervalles de temps prédéterminés respectivement, les images saisies sont évaluées quant à la couleur et/ou au contour des corps de produits en vrac et mémorisées sur un support de données, **caractérisé en ce que**
- les trois dispositifs d'éclairage (2.1, 2.2, 2.3) sont disposés dans un plan à la même hauteur par rapport à la zone de détection (1) de telle sorte qu'ils éclairent la zone de détection (1) à partir de différentes directions,
- un dispositif d'éclairage (2.3) formant une unité de construction avec l'au moins une caméra (4) parmi les trois dispositifs d'éclairage (2.1, 2.2, 2.3),
- le dispositif d'éclairage (2.3) formant une unité de construction avec la caméra (4) étant tourné vers l'élément de réflexion plat (3), et
- les deux autres dispositifs d'éclairage (2.1, 2.2) étant disposés en face l'un de l'autre par rapport à la zone de détection (1), de sorte qu'ils s'éclairent mutuellement.

2. Procédé de détection des corps étrangers selon la revendication 1, **caractérisé en ce que** le produit en vrac est éjecté lorsque les images détectées du produit en vrac montrent des corps étrangers différents quant à la couleur et/ou au contour des corps de produits en vrac.

3. Procédé de détection des corps étrangers selon la revendication 1, **caractérisé en ce que** du fromage râpé et revêtu d'un agent de séparation est utilisé comme produit en vrac.

4. Dispositif de détection des corps étrangers dans un flux de transport d'un produit en vrac tombant, présentant une zone de détection (1) pouvant être traversée par un flux de matériau tombant ainsi qu'au moins une caméra (4) orientée vers la zone de détection (1), **caractérisé en ce que**
la zone de détection (1) est bordée par trois dispositifs d'éclairage (2.1, 2.2, 2.3) et un élément de réflexion plat (3),
parmi les trois dispositifs d'éclairage (2.1, 2.2, 2.3), un dispositif d'éclairage (2.3) formant une unité de construction avec l'au moins une caméra (4), le dispositif d'éclairage (2.3) formant une unité de construction avec la caméra (4) comprenant un élément lumineux de forme plate qui est disposé sur une paroi de boîtier d'un boîtier (6) entourant l'au moins une caméra (4) ou qui fait partie de celui-ci, et le dispositif d'éclairage (2.3) formant une unité de construction avec la caméra (4) étant disposé sur la face avant du boîtier (6) tournée vers l'élément de réflexion plat (3), l'élément lumineux de forme plate présentant une zone transparente conçue comme fenêtre de caméra (7),
les trois dispositifs d'éclairage (2.1, 2.2, 2.3) étant disposés dans un plan à la même hauteur par rapport à la zone de détection (1) de telle sorte qu'ils éclairent la zone de détection (1) à partir de différentes directions,
l'au moins une caméra (4) reliée à un dispositif d'évaluation d'image (5) étant dirigée sur l'élément de réflexion (3) disposé derrière la zone de détection (1), l'élément de réflexion (3) de forme plate de l'au moins une caméra ainsi que le dispositif d'éclairage (2.3) formant une unité de construction avec la caméra (4) étant disposés en face l'un de l'autre par rapport à la zone de détection (1), et les deux autres dispositifs d'éclairage (2.1, 2.2) étant disposés en face l'un de l'autre par rapport à la zone de détection (1) de sorte qu'ils s'éclairent mutuellement,
le dispositif d'évaluation d'image (5) étant conçu pour générer un signal lorsqu'une couleur différente de la couleur du produit en vrac et/ou un contour différent du contour du produit en vrac apparaissent à l'intérieur d'une image saisie au moyen de l'au moins une caméra (4).

5. Dispositif de détection des corps étrangers selon la revendication 4, **caractérisé en ce que** l'élément de réflexion plat (3) présente une surface mate de la couleur du produit en vrac.

6. Dispositif de détection des corps étrangers selon la revendication 4, **caractérisé en ce qu'**un premier dispositif d'éclairage (2.1) et un deuxième dispositif d'éclairage (2.2) sont disposés en face l'un de l'autre par rapport à la zone de détection (1).

7. Dispositif de détection des corps étrangers selon la revendication 4, **caractérisé en ce qu'**il comporte au moins deux caméras (4), un angle entre les directions de visée des caméras (4) sur la zone de détection (1) étant au maximum de 30°, les caméras (4) et l'élément de réflexion (3) étant disposés en face l'un de l'autre par rapport à la zone de détection (1).

8. Dispositif de détection des corps étrangers selon la revendication 4, **caractérisé en ce qu'**il présente un dispositif de séparation disposé en amont de la zone de détection (1) dans le sens de transport du produit en vrac, avec lequel l'épaisseur du flux de transport peut être limitée.

9. Dispositif de détection des corps étrangers selon la revendication 4, **caractérisé en ce que** le boîtier (6) entourant l'au moins une caméra (4) est étanche à la poussière, ce boîtier pouvant être déplacé au moins sur un degré de liberté au moyen d'un dispositif de déplacement comprenant des bras de levier et des articulations.
